# EUROPEAN PATENT SPECIFICATION

(11) **EP 1 588 680 B1**
(45) Date of publication and mention of the grant of the patent: **28.11.2007**
(21) Application number: 05010738.2
(22) Date of filing: 04.05.1999
(51) Int. Cl.: A61F 6/08, A63B 23/20, A61B 5/107, A61H 21/00, A61F 2/00

(54) **Intravaginal set**
Intravaginaler Elementensatz
Jeu d'éléments intravaginaux

(30) Priority: 04.05.1998 PL 32614198
(43) Date of publication of application: 26.10.2005
(62) Divisional of application: 99201409.2
(73) Proprietor: Adamed SP. Z O.O., 05-152 Czosnów k/Warszawy (PL)
(72) Inventor: Adamkiewicz, Marian, deceased (PL)
(74) Representative: HOFFMANN EITLE

(56) References cited:
- DE-A- 2 121 790
- GB-A- 2 272 168
- US-A- 3 938 504
- US-A- 4 019 498
- US-A- 5 618 256

## Description

The present invention relates to an intravaginal set, used in the case of prolapse of the urogenital organs and urinary stress incontinence, or in the period of intervals, when a therapeutic intravaginal insert for treatment of static disorders of the urogenital organs and urinary stress incontinence is not currently inserted.

Aging and past parturitions result in weakening and elongation of the perineum muscles leading to the urogenital organs prolapse and other anatomical disorders. Once extended, muscles become weaker and weaker which subsequently results in progress of prolapse up to transvaginal eversion of the uterus. As the vaginal canal is the "locus minoris resistentiae" in the pelvic floor, walls of the vagina may become the ring of hernia (cystocele, uretrocele and rectocele) .

In the course of progressive prolapse of the urogenital organs, discomfort in lower abdominal part intensifies, from "heaviness" to pain, and urinary stress incontinence becomes apparent.

Prolapse of the urogenital organs causes decrease in distance between the uterine cervix and the vaginal inlet, descent of the anterior wall of the vagina along with the urinary bladder and urethra (the cysto-urethral angle becomes more obtuse), and urethra dislocates outside an operation range of the intra-abdominal pressure.

This results in impaired blood outflow from the urogenital organs due to venous constriction (low-pressure blood vessels).
In less advanced cases of the urogenital prolapse, special therapeutic exercises are recommended to strengthen the pelvic floor muscles. See also GB-A-2 272 168 for disclosure of a device for exercising the muscles of the pelvic floor that defines a cavity that contains a moving weight. See DE-A-2121790 for another such device

The physical exercises may increase the efficiency of the cross-striated muscles only, i.e. muscles dependent an our own will, whereas the urogenital organs equilibrium is also maintained by independent-of-our-will smooth muscles, controlled by the autonomous nervous system. Smooth muscles build the urinary bladder, urethra, internal sphincter muscle of urethra, and muscles in uterine ligaments fixing uterus in its normal position.

A significant progress in the treatment of static disorders of the urogenital organs has been made by development of a therapeutic insert (the subject of Polish patent specification no RP 138406), consisting of a hollow ball with string attached freely outside the ball, and the smaller metal ball, placed freely inside. This device generates mechanical impulses stimulating contractions of surrounding muscles, both smooth, and cross-striated. A metal ball placed freely in the intravaginal therapeutic insert generates the mechanical impulses by hitting its cap as a result of the centre of gravity translocation while walking. The mechanical impulse stimulates muscular contraction. Regular muscular exercise results in muscles hypertrophy and an increase in muscular force. The therapeutic insert may be applied twice a day, over the period of about 30 minutes.

Prolonged application longer than 30 minutes leads to muscular overstrain and abdominal pain. An application of the therapeutic insert every day over the period of 3 months leads to satisfactory therapeutic results in the treatment of minor prolapse of the urogenital organs (grade I), while in mere advanced cases (grades II, III, IV) no improvement was observed.

The most probable explanation is such, that in more advanced urogenital prolapse, the uterus lowers during intervals between applications of therapy, which results in unfavourable effects (isometric contraction, passive congestion).

When conservative therapy is ineffective, the reconstructive surgery is performed to restore the normal position of the urogenital organs.

Different strategies of therapeutic management are used, depending an symptoms, age and progress of the illness. From among around 200 modifications applied to correct the position of the urogenital organs, the basic management consists in shortening of muscles and ligaments. Such therapeutic management ameliorates the position, but it does not restore the normal muscular function and thus the lasting and complete recovery.

In the case of contraindications for surgery, the device prostheses may be applied to ensure a normal position of the urogenital organs.

One of them is well-known intravaginal disc for support of lowered or prolapsed uterus. This disc is applied in such manner, that its ring surrounds the uterine cervix, thus preventing the uterus prolapse by extension of vaginal wall in the area of the posterior vaginal fornix. A disadvantage of this design consists in difficulties in application and removal, what makes its use impossible, when the therapeutic insert is not currently used. As the disc is placed in the upper part of the vagina, it does not transmit the contraction of the levator ani muscle, thus the uterus is not elevated.

The respective British patent application discloses a plastic planar arc, the wider arm of which rests an the pubic symphysis, while the second, narrow arm presses the urethra against the urinary bladder. The maximal time for intravaginal application is 2 hours which limits the usefulness of this device in unlimited time. Mode of action consists in local compression of the urethra, what may lead to inflammation and decubitus ulcers.

There is also a well-known disposable vaginal pack (made in Germany), which is placed in the vagina over period up to 8 hours. Its mode of action consists in mild compression of urethra and adjustment to the vagina after soaking with water before intravaginal application. This vaginal pack, however, does not meet the corrective functions, and may cause inflammatory state in the case of prolonged intravaginal presence. Soaking with vaginal secretion may result in distension of the vagina leading to progressive prolapse of the uterus and urinary bladder.

Applicant's corresponding US Patent No. 6,530,879 includes a recitation of prior art which fulfils the duty of candour on the part of Applicant towards the United States Patent and Trademark Office. The list of prior publications include two papers by Morton et al which report on Applicant's own KOLPEXIN T (trademark) device that corresponds to the "therapeutic insert" described in the present specification. See US-A-4574791 for disclosure of a vaginal insert on a cable for training the pubo coccygeus muscle in a rhythmic contraction and relaxation that reciprocates the insert in the vaginal cavity.

The object of the present invention is to design an intravaginal set for a method of treatment to obtain permanent optimal position of the uterus and urinary bladder.

The invention is defined in claim 1 below. The dependent claims are directed to optimal and preferred features.

An intravaginal set according to the invention is useful in the case of prolapse of urogenital organs and urinary stress incontinence, or in the period of intervals in women when an intravaginal therapeutic insert for treatment of static disorders of the urogenital organs and urinary stress incontinence is not currently inserted, which therapeutic insert consists of hollow plastic ball with string attached and freely moving outside the ball, and with smaller ball, placed freely inside said hollow plastic ball, which smaller ball has a weight adequately adjusted to generate mechanical impulses stimulating alternate contractions of the smooth muscles, said intravaginal set comprising a sub-set of intravaginal corrective inserts and an intravaginal measuring sub-set for determining the size of the insert wherein the sub-set of intravaginal corrective inserts comprises at least two balls with step increase in diameter, ranging between the minimal and maximal woman vaginal diameter, each ball being preferably hollow, each ball has the loosely hanging string and each ball is made preferably of medical material, such as polycarbonate or methyl methacrylate, while the intravaginal measuring sub-set comprises at least two balls made of metal or plastic with graduated diameters corresponding to graduated diameters of the balls of sub-set of intravaginal corrective inserts, which measuring balls instead of the loosely hanging string, have a rigidly mounted, preferably linearly scaled slat for measurement of optimal diameter and depth of localization of the insert in the vagina, depending on actual and individual anatomical conditions of urogenital organs of the woman being treated.

The invention enables a method of treatment of prolapse of urogenital organs and urinary stress incontinence or in the period of intervals in women when the intravaginal therapeutic insert for treatment of static disorders of the urogenital organs and urinary stress incontinence is not currently inserted, by selecting the appropriate size of corrective insert from the sub-set of intravaginal corrective inserts, said selecting being realised by means of the intravaginal measuring sub-set, by the selection of appropriate optimal diameter and depth of localization of the corrective insert in vagina by approximations with using the balls from the measuring sub-set, so that contraction of the levator ani muscle will cause the elevation of the insert and the elevation of the insert will cause the elevation of the uterus and/or correction of the cysto-urethral angle, and during the progress of said treatment the sizes of successive applied corrective inserts are adjusted by analogic selection of appropriate optimal diameter and depth of localization of the measuring ball in the vagina, and advantageously by carrying out the exercises of the pelvic floor muscles of treated woman lying in prone genucubital position in the intervals between successive replacements of the corrective inserts.

An application of the corrective intravaginal insert enables maintaining of the insert in the vagina for an indefinite period of time. The insert diameter, selected by means of the measuring device, should ensure permanent correction of uterine and bladder placement, so that wall of the vagina was not too tense, and that a mass of lowered organs did not cause prolapse of the insert in the standing position.

Correction of the placement of the urogenital organs and reduction of the isometric contraction of muscles carrying urogenital organs result in restoration of normal blond supply, allowing them to regenerate and also prevent progressive lowering of the urogenital organs. Correction of the cysto-urethral angle enables control of the micturition.

The corrective insert from the set being the subject of the invention is used in the intervals between applications of the therapeutic insert and exerts beneficial effect on the muscles by removal of the isometric contraction and better preparation for dynamic contraction following application of the therapeutic insert. An alternating application of the therapeutic insert and the corrective insert enables the treatment of the more advanced cases of the urogenital prolapse and improves results of the therapy up to 80%.

The corrective insert applied alternately with the therapeutic insert, properly adjusted, supports the uterus in its optimal placement and, due to its spherical shape, it may move and turn freely in different directions, thus preventing, decubital ulceration.

The invention will now be described (by way of an illustrative example) in more detail with reference to the drawings, wherein Fig. 1 shows a sub-set of the corrective intravaginal inserts from the set being the subject of the invention, Fig. 2 shows the measuring sub-set from the set being the subject of the invention, Fig. 3 is a simplified sagittal cross-section of the female pelvis with the cysto-urethral angle enlarged almost up to 180°, when the micturition is started under control or in an involuntary way, Fig. 4 is a sagittal cross-section of the female pelvis with the corrective insert from the set being the subject of the invention causing correction of the cysto-urethral angle so that the resulting angle is right or obtuse; at this angle, the mechanism of urethral closure is most effective, and Fig, 5 is also a sagittal cross-section of the female pelvis and is showing a method of measuring the diameter of the vagina for determination of optimal localization and size of the corrective insert.

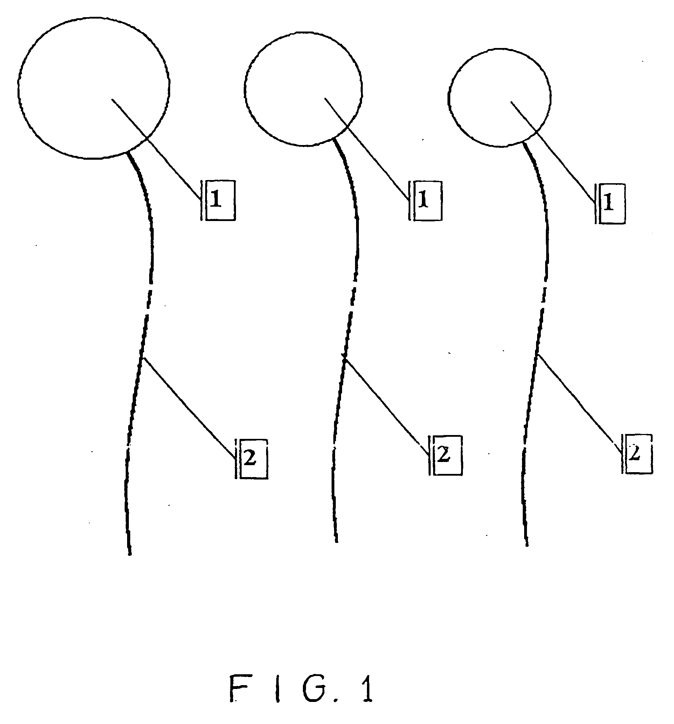

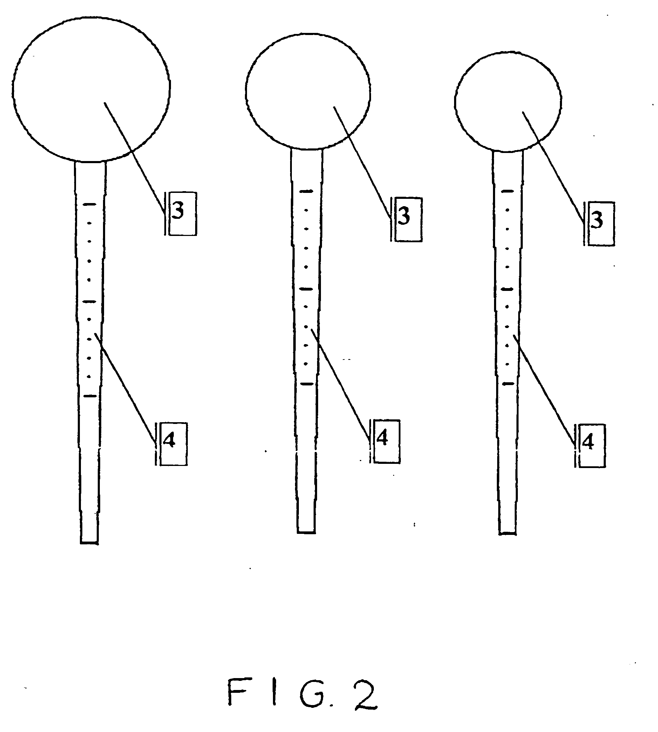

The intravaginal set being the subject of the invention consists of the sub-set of corrective intravaginal inserts (Fig. 1) being preferably the hollow balls 1 with step increase in diameter, ranging between the minimal and maximal vaginal diameter. Each ball 1 has the loosely hanging string 2 and is made preferably of medically inert plastics, such as polycarbonate or methyl methacrylate, and the set also consists of the intravaginal measuring sub-set (Fig. 2) containing measuring balls 3 made preferably of metal or plastic with graduated diameters corresponding to graduated diameters of balls 1 of the sub-set of corrective intravaginal inserts from Fig. 1 ; which balls 3 instead of the loosely hanging string 2 have, rigidly mounted, linearly scaled slat 4. The slat 4 is preferably scaled for measurement of diameter and optimal localization of the corrective insert in the vagina in the case of urinary stress incontinence. See WO 01/41880 for disclosure of an exercise device for insertion in the vagina, which has a shaft marked with measuring indicia.

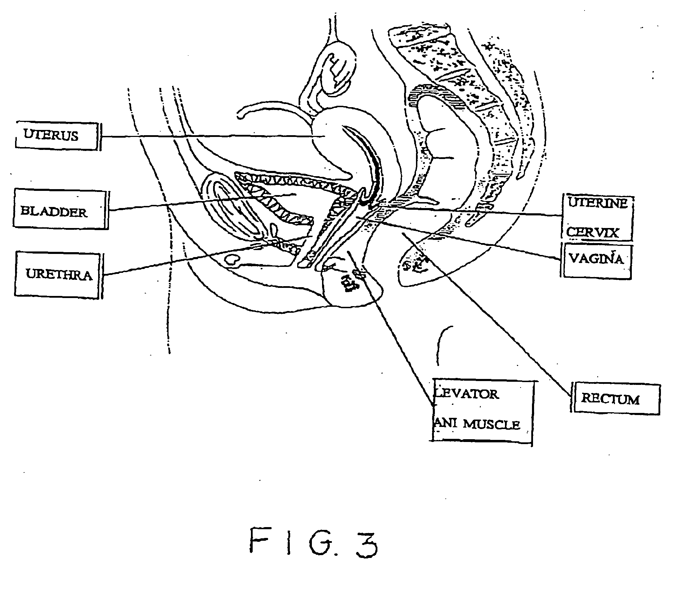

Fig. 3 shows a simplified sagittal cross-section of the female pelvis with the cysto-urethral angle enlarged almost up to 180°, when the micturition is controlled or involuntary.

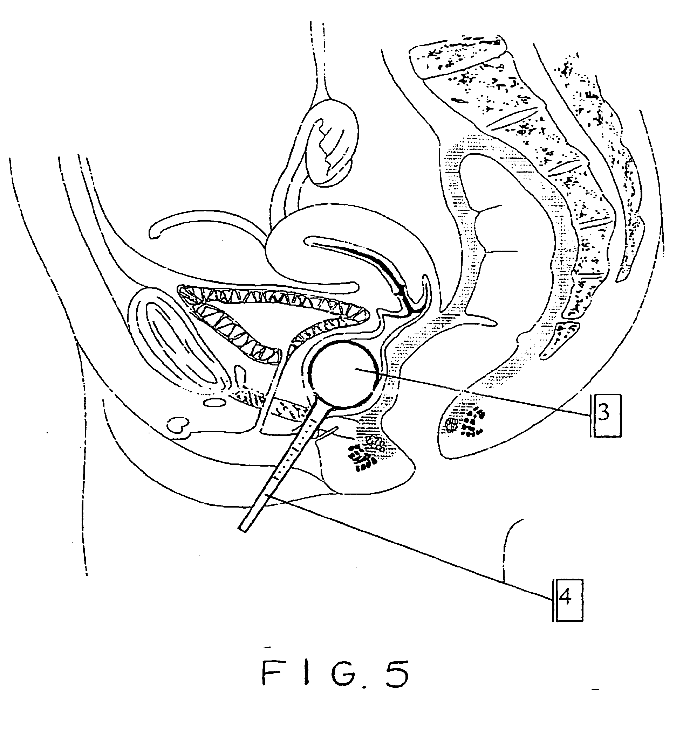

Fig. 5 shows the method of measuring diameter of the vagina using the ball 3 from the measuring sub-set for determination of depth of localization and size of the corrective insert. The appropriate anatomical size is adjusted using the balls 3 from the measuring sub-set. After the adjustment, it is checked, whether the measuring insert is not pushed out by the descending organs. In this case, a greater insert diameter should be applied. In the case of urgency, the insert is placed too high, then a smaller insert should be applied.

The application of the corrective intravaginal insert consists in determination of optimal diameter of the insert and intravaginal application of the corrective intravaginal insert for an indefinite period of time.

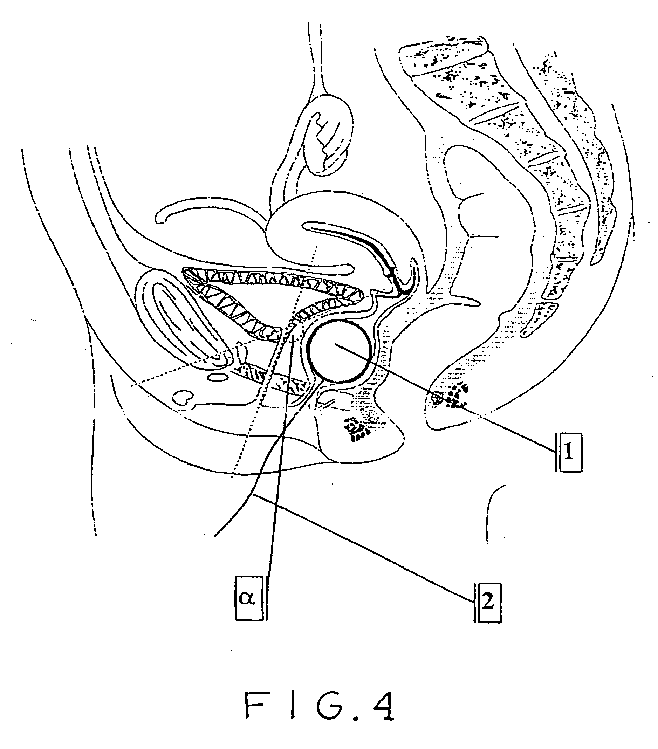

Fig. 4 shows a sagittal cross-section of the female pelvis with the corrective insert with properly selected diameter of the ball 1 by means of the measuring sub-set, and the string 2 is used for removal of the insert.

Mode of action of the insert is as follows: the insert being placed in the vagina is supported on the levator ani muscle. It results in forward and upward shift of the lowered anterior wall of the vagina and elevation of the urethra and urinary bladder. An elevation of the urethra restores its normal position in relation to the posterior wall of the urinary bladder (reduced cysto-urethral angle shown an Fig. 4). In the case of concomitant uterine prolapse, the insert causes elevation of the uterus.

An application of the corrective insert from the set being the subject of the invention results in very fast pain relief. The device also enables a controlled micturition due to correction of the cysto-urethral angle a without the necessity to remove the insert for micturition.

Displacement of the uterus to the "pure zone" enables complete removal of inflammation. Relaxation of muscles and fasciae restores normal blood supply of the organs of the pelvis minor. It is most probable that the corrective insert improves results of the treatment with the therapeutic insert. Alternating application of the therapeutic insert and the corrective insert enables the treatment of the more advanced cases of the urogenital descent and improves results of the therapy.

The insert can be easily applied and removed. It is supported an the posterior wall of the vagina at level of the levator muscle of anus, thus it prevents excessive extension of the vaginal walls.

The corrective insert from the set being the subject of the invention supports the prolapsed uterus and blocks the urethra in the case of urinary stress incontinence, inadequate urethral occlusion at increased intra-abdominal pressure. An optimal adjustment of insert diameter to the vagina allows the muscles to regenerate by reduction in uterine and cystic pressure. The corrective insert supports these organs, and after regeneration, the muscles will support the uterus and urinary bladder again, but in a corrected position.

A method of the treatment of prolapse of the urogenital organs and urinary stress incontinence in women is enabled with usage of the intravaginal set. What distinguishes this method is that the appropriate corrective insert is selected from the sub-set of corrective intravaginal inserts, consisting of at least two balls with step increase in diameter, ranging between the minimal and maximal vaginal diameter, each ball being preferably hollow, has the loosely hanging string and is made of medical material, such as polycarbonate or methyl methacrylate, and consisting of the intravaginal measuring sub-set that contains at least two metal or plastic balls with diameters corresponding to the diameters of the balls from corrective intravaginal sub-set which instead of the loosely hanging string, have, rigidly mounted, scaled slat for measurement of optimal diameter and localization of the insert in the vagina, depending on individual anatomical conditions of urogenital organ in woman, by selection of appropriate diameter and depth of vaginal localization by means of approximations with meaning balls from the measuring sub-set, so that contraction of the levator ani muscle causes elevation of the insert and elevation of the insert causes elevation of the uterus, and/or correction of the cysto-urethral angle, and during the progress of said treatment the sizes of successive applied inserts are adjusted by selection of appropriate diameter and depth of localization In the vagina and it is beneficial to carry out the exercises of the pelvic floor muscles laying on prone or genucubital position between the successive replacements of the insert.

Size of the applied insert gradually decreased under medical supervision using the measuring device and ensures permanent correction of uterine and bladder placement by correction of the cysto-urethral angle a, so that the muscle fibres diffused in surrounding tissue may restore the lowered organs to primary placement.

In the case of significant urogenital prolapse, the uterus is at the same level as the arms of the levator ani muscle, increasing distance between them.

In this case, an exercise of the levator ani muscle is disadvantageous, because muscular contraction exerts pressure on the descendent uterus without concomitant elevation. In this case the corrective intravaginal insert causes an elevation of the uterus, while contractions of the levator ani muscle elevates the insert and, indirectly, the uterus. The corrective insert may fall out at muscular contraction, when its diameter is too small. It is beneficial to make the test for contraction of the levator ani muscle following an adjustment of the corrective insert by the measuring device.

An exercise of the levator ani muscle with the usage of the corrective insert leads to improved efficiency of the levator ani muscle, vaginal stenosis and elevation of the uterus.

It is necessary to check position of the insert and the uterus within 2 weeks from the beginning of the recommended exercise.

In advanced cases of urogenital prolapse in which the uterine cervix is placed below the arms of the levator ani muscle, an exercise of the muscle with the usage of the corrective insert elevates the uterus with decrease in distance between both arms; the insert elevates, resulting in uterine elevation.

Progress in therapy may be monitored using the measuring device for examination of so-called pelvic floor thickness, i.e. following application, the measuring device is slightly pulled downward to rest it on the arms of the levator ani muscle and the distance from the pubic symphysis is read from the scaled slat.

An assessment of therapeutic effect includes repeated measurements of optimal diameter of the corrective insert.

The numbered paragraphs which follow will reveal further aspects of the present invention.
1. An intravaginal set, used in the case of prolapse of urogenital organs and urinary stress incontinence, or in the period of intervals in women when the intravaginal therapeutic insert for treatment of static disorders of the urogenital organs and urinary stress incontinence is not currently inserted, which therapeutic insert consists of hollow plastic ball with string attached and freely moving outside the ball, and with smaller ball, placed freely inside said hollow plastic ball, which smaller ball has a weight adequately adjusted to generate mechanical impulses stimulating alternate contractions of the smooth muscles, said set comprising a subset of intravaginal corrective inserts and an intravaginal measuring subset for determining the size of the insert; wherein the subset of intravaginal corrective inserts consists of at least two balls (1) with step increase in diameter, ranging between the minimal and maximal woman vaginal diameter, each ball having means (2) for removing it from a vagina, while the intravaginal measuring subset comprises at least two balls (3) with graduated diameters corresponding to graduated diameters of the balls (1) of subset of intravaginal corrective inserts, which measuring balls (3) instead of the removing means (2), have means (4) for measurement of optimal diameter and depth of localization of the insert in the vagina, depending on actual and individual anatomical conditions of urogenital organs of the woman being treated.
2. The intravaginal set as in paragraph 1, wherein each ball (1) of the subset of corrective inserts is hollow.
3. The intravaginal set as in paragraph 1 or 2, wherein the means for removing each ball (1) of the subset of corrective inserts from the vagina comprise a loosely hanging string (2).
4. The intravaginal set as in any one of the preceding numbered paragraphs, wherein each ball (1) of the subset of corrective inserts is made from a medical material such as polycarbonate.or methyl methacrylate.
5. The intravaginal set as in any one of the preceding numbered paragraphs, wherein each ball (3) of the measuring subset is made of metal or plastic.
6. The intravaginal set as in any one of the preceding numbered paragraphs, wherein the measurement means comprise a slat (4) rigidly mounted on each ball (3) of the measuring subset.
7. The intravaginal set as in paragraph 6, wherein the slat (4) has a linear measuring scale.
8. A method of treatment of prolapse of urogenital organs and urinary stress incontinence or in the period of intervals in women when the intravaginal therapeutic insert for treatment of static disorders of the urogenital organs and urinary stress incontinence is not currently inserted, said method being realized by means of an intravaginal set of inserts, and by the steps of selecting the appropriate size of corrective insert from a subset of intravaginal corrective inserts, consisting of at least two balls with step increase in diameter, ranging between the minimal and maximal woman vaginal diameter, each ball being preferably hollow, each ball has the loosely hanging string and each ball is made preferably of medical material, such as polycarbonate or methyl methacrylate, said selecting being released by means of an intravaginal measuring subset comprising at least two metal or plastic balls having graduated diameters corresponding to graduated diameters of the balls from the subset of the intravaginal corrective inserts, which balls instead of the loosely hanging string have a rigidly mounted linearly scaled slat for measurement of optimal diameter and depth of localization of the corrective insert in the vagina, depending on actual and individual anatomical conditions of urogenital organ of the woman being treated, by the selection of appropriate optimal diameter and depth of localization of the corrective insert in vagina by approximations with using the balls from the measuring subset, so that contraction of the levator ani muscle will cause the elevation of the insert and the elevation of the insert will cause the elevation of the uterus and/or correction of the cysto-urethral angle, and during the progress of said treatment the sizes of successive applied corrective inserts are adjusted by analogic selection of appropriate optimal diameter and depth of localization of the measuring ball in the vagina, and advantageously by carrying out the exercises of the pelvic floor muscles of treated women lying in prone or genucubital position in the intervals between successive replacement of the corrective inserts.
9. An application of an intravaginal set in the treatment of prolapse of urogenital organs and urinary stress incontinence or in the period of intervals in women when the intravaginal therapeutic insert for treatment of static disorders of the urogenital organs and urinary stress incontinence is not currently inserted, said method being realized by means of an intravaginal set of inserts, and by the steps of selecting the appropriate size of corrective insert from a subset of intravaginal corrective inserts, consisting of at least two balls with step increase in diameter, ranging between the minimal and maximal woman vaginal diameter, each ball being preferably hollow, each ball has the loosely hanging string and each ball is made preferably of medical material, such as polycarbonate of methyl methacrylate, said selecting being realized by means of an intravaginal measuring subset comprising at least two metal or plastic balls having graduated diameters corresponding to graduated diameters of the balls from the subset of the intravaginal corrective inserts, which balls instead of the loosely hanging string have a rigidly mounted linearly scaled slat for measurement of optimal diameter and depth of localization of the corrective insert in the vagina, depending on actual and individual anatomical conditions of urogenital organ of the woman being treated, by the selection of appropriate optimal diameter and depth of localization of the corrective insert in vagina by approximations with using the balls from the measuring subset, so that contraction of the levator ani muscle will cause the elevation of the insert and the elevation of the insert will cause the elevation of the uterus and/or correction of the cysto-urethral angle, and during the progress of said treatment the sizes of successive applied corrective inserts are adjusted by analogic selection of appropriate optimal diameter and depth of localization of the measuring ball in the vagina, and advantageously by carrying out the exercises of the pelvic floor muscles of treated woman lying in prone or genucubital position in the intervals between successive replacements of the corrective inserts.

## Claims

1. An intravaginal set for treating disorder of a urogenital organ, the set comprising a first sub-set of corrective ball inserts and a second sub-set of measuring balls, with each corrective ball insert having a counterpart measuring ball of the same diameter, each sub-set having a plurality of balls of stepwise different diameter, and wherein each of said balls of said first sub-set has a string attached thereto to permit the ball to be extracted from the vagina by pulling on the string, and so to assist in removal of the ball from the body;
each ball of the corrective sub-set of inserts having inside no freely-moving weight, whereby the insert may remain inserted for an indefinite period of time in order to elevate said urogenital organ and so relieve pain during the period of insertion; and
each ball of the measuring sub-set having extending therefrom a slat, by which a physician can select an appropriate diameter for the corrective insert.

2. Device as claimed in claim 1, wherein each said slat exhibits a measuring scale strip to determine location of the ball in the body cavity.

3. An intravaginal device as in claim 1 or 2, wherein each of said balls of said first sub-set is of plastic.

4. An intravaginal device as in claim any one of the preceding claims, wherein said balls of said second sub-set are of plastic or metal.

## Patentansprüche

1. Intravaginales Set zum Behandeln von einer Störung eines urogenitalen Organs, wobei das Set ein erstes Teilset aus korrigierenden Kugeleinsätzen und ein zweites Teilset aus Messkugeln umfasst, wobei jeder korrektive Kugeleinsatz eine entsprechende Messkugel desselben Durchmessers aufweist, wobei jeder Teilsatz eine Vielzahl von Kugeln mit schrittweise unterschiedlichem Durchmesser aufweist und wobei jede der Kugeln des ersten Teilsets einen daran befestigten Faden aufweist, um es der Kugel zu erlauben, durch Ziehen an dem Faden aus der Vagina herausgezogen zu werden und so bei der Entfernung der Kugel aus dem Körper zu helfen;
wobei jede Kugel des korrektiven Teilsets der Einsätze im Inneren kein frei bewegliches Gewicht aufweist, wobei der Einsatz für eine unbestimmte Zeitdauer eingesetzt verbleiben kann, um das urogenitale Organ anzuheben und Schmerzen während des Zeitraums des Einsetzens zu lindern; und
wobei jede Kugel des Messteilsets einen sich daraus erstreckenden Streifen aufweist, mithilfe dessen ein Arzt einen geeigneten Durchmesser für den korrektiven Einsatz auswählen kann.

2. Vorrichtung nach Anspruch 1, wobei jeder Streifen einen Messskalastreifen aufweist, um die Position der Kugel in dem Körperhohlraum zu bestimmen.

3. Intravaginale Vorrichtung nach Anspruch 1 oder 2, wobei jede der Kugeln des ersten Teilsets aus Kunststoff besteht.

4. Intravaginale Vorrichtung nach einem der vorangehenden Ansprüche, wobei die Kugeln des zweiten Teilsets aus Kunststoff oder Metall bestehen.

## Revendications

1. Ensemble intravaginal destiné à traiter un trouble d'un organe urogénital, l'ensemble comprenant un premier sous-ensemble d'inserts de balles correctifs et un deuxième sous-ensemble de balles de mesure, avec chaque insert de balle correctif ayant une balle de mesure en contrepartie du même diamètre, chaque sous-ensemble ayant une pluralité de balles de diamètre progressivement différent, et dans lequel chacune desdites balles dudit premier sous-ensemble a un cordon étant attaché à celle-ci pour permettre à la balle d'être extraite du vagin en tirant sur le cordon, et ainsi aider au retrait de la balle du corps ;
chaque balle du sous-ensemble correctif d'inserts n'ayant à l'intérieur aucun poids se déplaçant librement, d'où il résulte que l'insert peut rester inséré pendant une période indéterminée afin d'élever ledit organe urogénital et de soulager ainsi une douleur pendant la période d'insertion ; et
chaque balle du sous-ensemble de mesure ayant une lame s'étendant depuis celle-ci, par laquelle un médecin peut choisir un diamètre approprié pour l'insert correctif.

2. Dispositif selon la revendication 1, dans lequel chacune desdites lames présente une bande d'échelle de mesure pour déterminer l'emplacement de la balle dans la cavité corporelle.

3. Dispositif intravaginal selon la revendication 1 ou 2, dans lequel chacune desdites balles dudit premier sous-ensemble est en plastique.

4. Dispositif intravaginal selon l'une quelconque des revendications précédentes, dans lequel lesdites balles dudit deuxième sous-ensemble sont en plastique ou en métal.
